Europäisches Patentamt

European Patent Office

Office européen des brevets

(19)

(11) Veröffentlichungsnummer: **0 383 316 B1**

(12) **EUROPÄISCHE PATENTSCHRIFT**

(45) Veröffentlichungstag der Patentschrift: **02.02.94**

(51) Int. Cl.⁵: **C07B 57/00**, C07D 405/04

(21) Anmeldenummer: **90102917.3**

(22) Anmeldetag: **15.02.90**

(54) **Verfahren zur Enantiomerentrennung eines Benszopyranderivats.**

(30) Priorität: **15.02.89 DE 3904496**

(43) Veröffentlichungstag der Anmeldung:
**22.08.90 Patentblatt 90/34**

(45) Bekanntmachung des Hinweises auf die
Patenterteilung:
**02.02.94 Patentblatt 94/05**

(84) Benannte Vertragsstaaten:
**AT BE CH DE DK ES FR GB GR IT LI LU NL SE**

(56) Entgegenhaltungen:
**EP-A- 0 273 262**
**DE-A- 1 948 368**

**JOURNAL OF MEDICINAL CHEMISTRY, Band
29, Nr. 11, November 1986, Seiten 2194-2201,
Washington, DC, US; V.A. ASHWOOD et al.:
"Synthesis and antihypertensive activity of
4-(cyclic amido)-2H-1-benzopyrans"**

(73) Patentinhaber: **MERCK PATENT GmbH
Postfach,
Frankfurter Strasse 250
D-64271 Darmstadt(DE)**

(72) Erfinder: **Devant, Ralf M., Dr.
Im Hilfsbruch 87
D-6100 Darmstadt-Arheilgen(DE)**

## Beschreibung

Die Erfindung betrifft ein Verfahren zur Enantiomerentrennung von trans-3-Hydroxy-3,4-dihydro-2,2-dimethyl-4-(2-oxo-1,2-dihydropyridin-1-yl)-2H-1-benzopyran-6-carbonitril [trans-2,2-Dimethyl-4-(2-pyridon-1-yl)-6-cyan-chroman-3-ol; "I"].

Racemisches I, ein Konglomerat der beiden Enantiomeren, ist aus der EP-A-0 273 262 bekannt. Es finden sich dort auch Angaben, wonach man Verbindungen einer bestimmten allgemeinen Formel, die auch die Verbindung I umschließt, nach an sich bekannten Methoden in ihre Enantiomeren trennen kann. Nähere experimentelle Einzelheiten zur Herstellung der Enantiomeren (+)-I und (-)-I aus I sind dort jedoch nicht beschrieben.

Es ist möglich, I durch Derivatisierung mit chiralen Reagenzien und anschließende fraktionierte Kristallisation zu trennen. Insbesondere kann I mit chiralen Isocyanaten zu den entsprechenden Urethanen umgesetzt werden, z.B. mit (+)- bzw. (-)-1-Phenylethylisocyanat zu den entsprechenden 1-Phenylethylurethanen; diese können anschließend fraktioniert kristallisiert und die jeweils erhaltenen beiden Diastereomeren dann hydrolysiert werden.

Diese Verfahren der chemischen Enantiomerentrennung haben in der Praxis jedoch große Nachteile. So sind teure Hilfsreagenzien erforderlich; die Trennung erfordert zwei zusätzliche chemische Umsetzungen.

Der Erfindung liegt die Aufgabe zugrunde, ein verfahren zur Enantiomerentrennung von I zur Verfügung zu stellen, das die Nachteile dieser Verfahren nicht oder nur in geringerem Maße besitzt. Diese Aufgabe wurde durch die Auffindung des vorliegenden Verfahrens der "Cristallization by Entrainment" gelöst.

Gegenstand der Erfindung ist demnach ein Verfahren zur Enantiomerentrennung von I, dadurch gekennzeichnet, daß man racemisches I zusammen mit einer geringen Menge (-)-I [bzw. (+)-I] in einem Gemisch von Dichlormethan mit einem niederen Alkohol löst, die Lösung mit (-)-I [bzw. (+)-I] animpft, das ausgefallene (-)-I [bzw. (+)-I] isoliert, in dem Filtrat weiteres racemisches I löst, mit (+)-I [bzw. (-)-I] animpft, das ausgefallene (+)-I [bzw. (-)-I] isoliert und gewünschtenfalls diesen Kristallisationszyklus ein oder mehrere Male wiederholt.

Es ist überraschend, daß dieses Entrainment-Verfahren im Falle von I mit Erfolg angewendet werden kann. So gelangen Enantiomerentrennungen verwandter Verbindungen, z.B. des 3-Methylderivats von I, nicht.

Als Lösungsmittel eignen sich vorzugsweise Gemische von halogenierten Kohlenwasserstoffen, insbesondere Dichlormethan, mit niederen Alkoholen, die 1-4 C-Atome enthalten, insbesondere Methanol, Ethanol oder Isopropanol. Bevorzugt sind Gemische, die Dichlormethan und Ethanol im Volumenverhältnis von 10:1 bis 30:1, insbesondere 20:1 enthalten.

Im einzelnen löst man racemisches I zusammen mit etwa 1,5-2,5 Gew.% (-)- oder (+)-I zweckmäßig in der Hitze in einem Gemisch von etwa 30-50 Volumina (z.B. ml, bezogen auf 1 g I) Dichlormethan und 1,5-2,5 Volumina Ethanol, kühlt ab und impft mit etwa 0,1-0,3 Gew.% reinem (-)-I [oder (+)-I]. Das auskristallisierte (-)-I [oder (+)-I] wird isoliert, zweckmäßig abfiltriert. Das Filtrat wird zweckmäßig mit einer weiteren Menge des Racemats versetzt, die der Menge des vorher abfiltrierten Enantiomeren entspricht, und das zugesetzte Material wird in der Wärme gelöst. Erneutes Abkühlen und Animpfen mit (+)-I [oder (-)-I], also dem anderen Enantiomeren, bewirkt eine Auskristallisation von (+)-I [oder (-)-I], welches man ebenfalls isoliert, zweckmäßig abfiltriert. Dieser Kristallisationszyklus kann ein oder mehrere Male wiederholt werden, indem man in dem zuletzt erhaltenen Filtrat weiteres racemisches I auflöst, durch Abkühlen und Animpfen weiteres (-)-I [oder (+)-I] zur Kristallisation bringt, im Filtrat davon erneut racemisches I auflöst, durch Abkühlen und Animpfen weiteres (+)-I [oder (-)-I] gewinnt usw.

Beispiel

In einem siedenden Gemisch von 2 l Dichlormethan und 100 ml Ethanol werden 49 g racemisches I und 1 g reines (-)-I gelöst. Unter Rühren läßt man auf 20° abkühlen und impft mit 100 mg reinem (-)-I. Nach 2 Std. werden 7 g (-)-I abfiltriert; optische Reinheit 96 % ee.

Zur Mutterlauge werden 7 g racemisches I zugegeben und durch Kochen gelöst. Nach dem Abkühlen auf 20° impft man mit 100 mg reinem (+)-I. Nach 2 Std. werden 6 g (+)-I abfiltriert; optische Reinheit 93 % ee.

Der Kristallisationszyklus läßt sich mehrere Male wiederholen.

Durch nochmaliges Umkristallisieren der so erhaltenen Enantiomere aus Ethanol oder Dichlormethan/Ethanol-Gemischen erhält man Produkte mit einer Enantiomerenreinheit von ≥ 99 % ee:

(-)-Enantiomer, F. 262-263°; $[\alpha]_D^{20}$ -88,5 ° (c = 1 in Methanol)

(+)-Enantiomer, F. 262-263°; $[\alpha]_D^{20}$ +87,8 ° (c = 1 in Methanol)

Die optische Reinheit wird wie folgt bestimmt:

Man löst 2,5 mg I in 2 ml trockenem Tetrahydrofuran. Nach Zugabe von 30 $\mu$l 1,8-Diazabicyclo-[5,4,0]undecen-(7) und 10 $\mu$l (R)-(+)-1-Phenylethylisocyanat rührt man 2 Std. bei 20°, nimmt anschließend in Ethylacetat auf und wäscht mit NaHCO$_3$-Lösung und Wasser. Nach Trocknung und Entfernen des Lösungsmittels werden die Diastereomere HPLC-chromatographisch (Säule: Merck RP-18; Laufmittel: Wasser/Acetonitril 65:35) analysiert.

**Patentansprüche**

**1.** Verfahren zur Enantiomerentrennung von trans-3-Hydroxy-3,4-dihydro-2,2-dimethyl-4-(2-oxo-1,2-dihydropyridin-1-yl)-2H-1-benzopyran-6-carbonitril (I), dadurch gekennzeichnet, daß man racemisches I zusammen mit einer geringen Menge (-)-I [bzw. (+)-I] in einem Gemisch von Dichlormethan mit einem niederen Alkohol löst, die Lösung mit (-)-I [bwz. (+)-I] animpft, das ausgefallene (-)-I [bzw. (+)-I] isoliert, in dem Filtrat weiteres racemisches I löst, mit (+)-I [bzw. (-)-I] animpft, das ausgefallene (+)-I [bzw. (-)-I] isoliert und gewünschtenfalls diesen Kristallisationszyklus ein oder mehrere Male wiederholt.

**Claims**

**1.** Process for resolving the enantiomers of trans-3-hydroxy-3,4-dihydro-2,2-dimethyl-4-(2-oxo-1,2-dihydropyridin-1-yl)-2H-1-benzopyran-6-carbonitrile (I), characterized in that racemic I, together with a small amount of (-)-I [or (+)-I], is dissolved in a mixture of methylene chloride with a lower alcohol, the solution is seeded with (-)-I (or (+)-I], the (-)-I [or (+)-I] which has precipitated is isolated, further racemic I is dissolved in the filtrate, the mixture is seeded with (+)-I [or (-)-I], the (+)-I [or (-)-I] which has precipitated is isolated, and if desired this crystallization cycle is repeated once or several times.

**Revendications**

**1.** Procédé de fractionnement d'énantiomères de trans-3-hydroxy-3,4-dihydro-2,2-diméthyl-4-(2-oxo-1,2-dihydropyridin-1-yl)-2H-1-benzopyrane-6-carbonitrile (1), caractérisé en ce qu'on dissout le racémique I avec une faible quantité (-)-I [ou (+)-I] dans un mélange de dichlorométhane et d'un alcool inférieur, on ensemence la solution avec (-)-I [ou (+)-I], on isole le précipité de (-)-I [ou (+)-I], dans le filtrat on dissout du racémique I supplémentaire, on ensemence avec (+)-I [ou (-)-I], on isole le précipité de (+)-I [ou (-)-I] et si c'est souhaité on répète ce cycle de cristallisation une ou plusieurs fois.